# EUROPEAN PATENT SPECIFICATION

(11) **EP 3 653 244 B1**
(45) Date of publication and mention of the grant of the patent: **29.12.2021**
(21) Application number: 18206152.3
(22) Date of filing: 14.11.2018
(51) Int. Cl.: A61M 5/32

(54) **A DEVICE FOR MOUNTING A SYRINGE NEEDLE TO A SYRINGE BARREL**
VORRICHTUNG ZUM ANBRINGEN EINER SPRITZENNADEL AN EINEM SPRITZENZYLINDER
DISPOSITIF DE MONTAGE D'UNE AIGUILLE DE SERINGUE SUR UN CYLINDRE DE SERINGUE

(43) Date of publication of application: 20.05.2020
(73) Proprietor: Galderma S.A., 6330 Cham (CH)
(72) Inventor: KARLSSON, Henrik, 749 52 Grillby (SE); TYRSING, Oliver, 752 39 Uppsala (SE)
(74) Representative: AWA Sweden AB

(56) References cited:
- WO-A1-2015/085031
- JP-A- 2015 150 241
- US-A1- 2005 271 465

## Description

### Technical field

The present invention relates to a device for enabling easier and more secure assembly of a needle or a cannula to a syringe barrel.

### Background

Syringes are widely used and come in many sizes and shapes. Typically, a syringe has a syringe barrel and a separate needle attachable to the syringe barrel by means of a threaded connection. The needle comprises a needle hub provided with connecting geometries of the threaded connection of the needle. A widely used type of threaded connection is the so called Luer taper. During assembly of the threaded connection it is important that an adequate momentum is applied between needle hub and syringe barrel. Too high momentum risks breaking the involved parts and prevents disassembly. Too low momentum risks leading to leaks between the needle hub and the syringe barrel. Hence, there is a need for an improved device allowing easy and proper connection between needle hub and syringe barrel.

### Summary

An object of the invention is to provide for easy and secure connection between a needle hub and a syringe. According to a first aspect, at least this object is achieved with a torque device for mounting of a syringe needle to a syringe barrel as defined in the appended claims. The torque device comprises a device hub and a torque ring. The device hub is provided with a central recess extending along a rotational axis of the device hub, wherein said central recess is configured to receive and grab a needle hub of a syringe needle, directly or via a needle cover attached to the needle hub, and with the needle extending along the rotational axis of the device hub. Also, the torque ring is provided with a plurality of arms extending inwards from the torque ring, wherein the arms together define a central space within which the device hub is rotatable. The device hub is provided with a plurality of abutment portions configured to engage respective inner end portions of the respective arms upon relative rotation between the device hub and the torque ring such that a momentum applied to the torque ring is transferred to the device hub via the engagement between the arms and the abutment portions. The arms and the abutment portions are configured so that the arms bend and slip over the abutment portions upon application of momentum over a predetermined threshold level at rotation of the torque ring in a tightening direction for mounting of the needle hub to the syringe barrel.

The torque ring enables a user to grab the torque device and apply momentum to the needle hub with little effort and improved control. The device hub holds the needle hub directly or indirectly via a needle cover attached to the needle hub, with the needle hub or cover in its central recess while the needle hub is screwed onto the tip of the syringe barrel by application of torque to the needle hub directly or, if the needle cover is attached to the needle hub, indirectly via the needle cover. The arms transfer torque from the torque ring to the needle hub whilst limiting the maximum momentum transferable to the needle hub by the resiliency of the arm allowing them to slip past the abutment portions once the torque applied exceeds a predetermined threshold level.

The plurality of arms may comprise at least three arms radially separated around the torque ring. By providing at least three arms radially distributed around the torque ring, the arms support the device hub from at least three directions, thereby preventing the device hub from leaving the central space upon operation of the torque device.

The device hub may be provided with a circumferential flange extending radially outwards around the rotational axis of the device hub, wherein the plurality of arms engages the flange from both axial sides of the flange. The provision of arms engaging the flange from both axial sides mitigates axial relative displacement between the torque ring and the device hub and assists the user in correct operation of the torque device.

The device hub may be larger than the central space provided by the arms, wherein the arms of the torque ring bend slightly when the device hub is mounted in the torque ring. Hence, the arms are normally biased towards the device hub, which ensures a proper fit between device hub and torque ring although the exact size of the device hub may vary slightly. Further, since the arms are normally biased, the power required for the arms to pass the abutment portions is more even throughout the relative rotational movement of the device hub and the torque ring.

The plurality of arms may comprise a first and a second arm provided on radially opposing sides of the device hub with respect to each other, both on a first axial side of the circumferential flange. Also, the plurality of arms may comprise a third and a fourth arm provided on radially opposing sides of the device hub with respect to each other, both on a second axial side of the flange, said second axial side being opposite the first axial side. The first and second arm together define a first pivot axis for the flange, wherein the third and fourth arm together define a second pivot axis for the flange. Also, the first, second, third and fourth arms are positioned such that the first and second axes are not parallel. The first and second arms block movement of the flange in a first axial direction along the rotational axis whilst the third and fourth arms block movement of the flange in a second axial direction opposite the first axial direction. Since the first and second axes are not parallel, any rotation of the device hub around the first axis is prevented by the third and fourth arms and vice versa. This provides for a very simple and robust technical solution allowing the device hub to be easily inserted into the central space upon manufacturing of the torque device.

At least one of the arms is attached to the torque ring backward or forward of the normal of the torque ring through the tip of the arm. By so directing the arm, the tip of the arm moves more radially outward upon bending of the arm as compared to if the arm would be directed straight along the radial direction of the needle hub. The increased tendency of the tip to move radially outward upon being forced onto the abutment portions at application of torque on the torque ring, provides for a more distinct click when the arm passes the abutment portions; This, since only a little relative rotation is required to move the tip of the arm radially outward enough for the abutment portions to be able to slide under and past the arm.

At least some of the abutment portions may comprise a leading surface angled in the range of 20-130 degrees relative to the opening direction of the respective arm at contact with the respective arm upon application of torque on the torque ring for tensioning of the needle to the syringe barrel.

When the leading surface is so oriented, the tip of the arm is forced in it's opening direction by the leading surface upon application of increased torque for tensioning of the needle hub to the syringe barrel.

### Brief description of the drawings

Figs. 1-10 all show views of a first embodiment of the torque device.
Figs. 1-5 show views of the torque device upon rotation in a tightening direction as indicated by reference numeral 11.
Figs. 6-8 show views of the torque device upon rotation in a loosening direction opposite to the tightening direction as indicated by reference numeral 20.

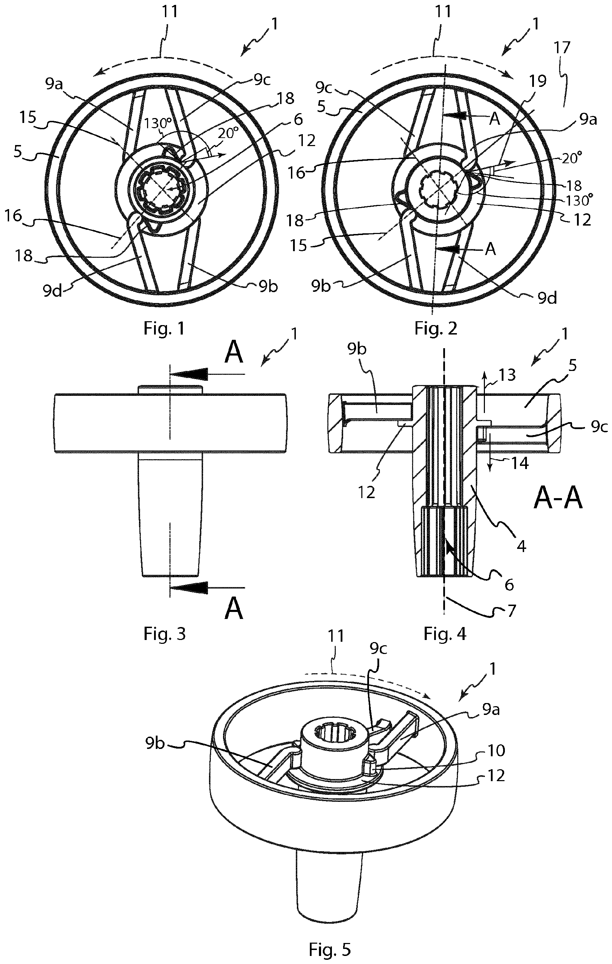

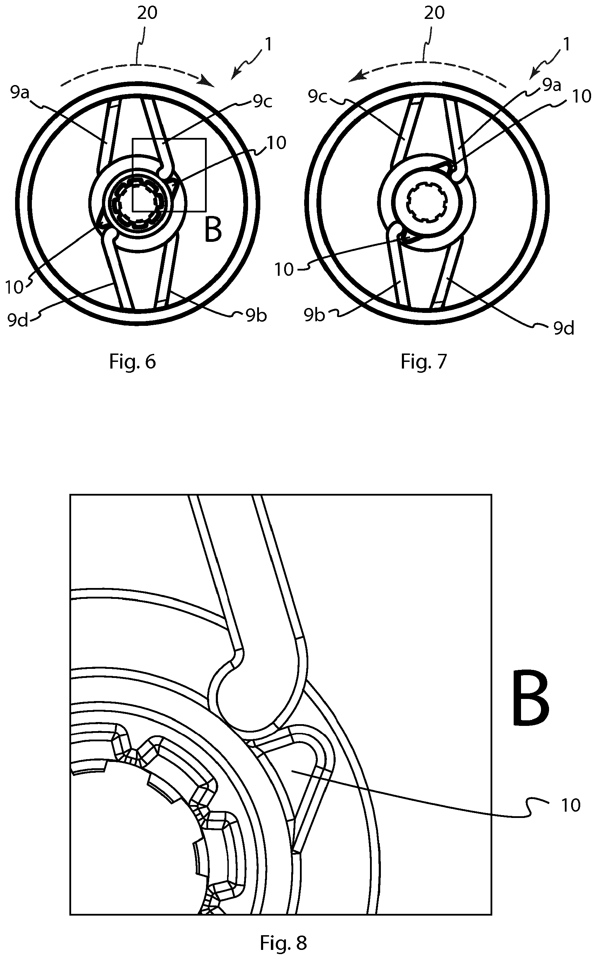

Specifically, Figs. 1 and 6 are views from below (as seen from the syringe), whilst Figs. 2 and 7 are views from above as seen from a user of the tool upon operation of the tool.

Fig. 3 is a side view and Fig. 4 is a sectional view in section A-A as indicated in Figs. 2 and 3.

Fig. 5 is a perspective view from above of the torque device.

Fig. 8 is an enlarged detail view of portion B as indicated in Fig. 6. This view shows how the arms firmly engage the abutment portions upon loosening operation to allow unrestricted force to loosen the needle hub. The arms 9c-d on the bottom side of the torque device 1 cannot slide past the respective abutment portions 10 upon rotation in the loosening direction 20.

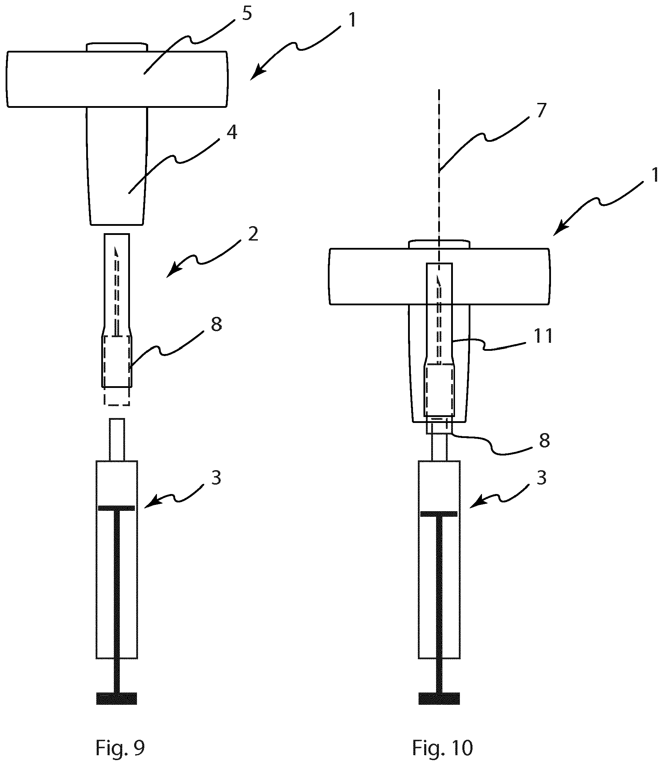

Fig. 9 is a a side view of the torque device and the syringe before attaching the needle hub to the syringe barrel by applying momentum to the needle cover of the needle hub.

Fig. 10 corresponds to Fig. 9 but shows the torque device in use with the needle hub positioned in the torque device with a needle cover attached to the needle hub.

Note that Figs. 9 and 10 merely provide schematic illustrations of the syringe and that actual dimensions and shapes may vary.

| | | | |
|---|---|---|---|
| 1 | torque tool | 12 | circumferential flange |
| 2 | syringe needle | 13 | first axial side of flange |
| 3 | syringe barrel | 14 | second axial side fo flange |
| 4 | tool hub | 15 | first pivot axis |
| 5 | torque ring | 16 | second pivot axis |
| 6 | central recess | 17 | normal of the torque ring (through tip of arm) |
| 7 | rotational axis of tool hub | 18 | leading surface |
| 8 | needle hub of syringe needle | 19 | opening direction of arm |
| 9 | arms | 20 | loosening direction |
| 10 | abutment means | 21 | needle cover |
| 11 | tightening direction | | |

### Detailed description of embodiments

Embodiments of the torque device will hereinafter be described with reference to the appended drawings.

According to a first embodiment, the torque device 1 comprises a device hub 4 and a torque ring 5. The device hub 4 is provided with a central recess 6 extending along a rotational axis 7 of the device hub 4. The central recess 6 is configured to receive and grab a needle hub 8 of a syringe needle 2 with the needle 2 extending along the rotational axis 7 of the device hub 4. The central recess 6 is in this embodiment a substantially cylindrical recess sized to be able to receive a standard size needle hub. The cylindrical recess is provided with a plurality of protrusions extending radially inwards from the inside of the cylindrical recess in order to interfere and grab hold of edges of the needle hub 8 to thereby prevent relative rotation between the needle hub 8 and the device hub 4 at application of torque for screwing the needle hub 8 onto the syringe barrel 3 upon use of the torque device 1. Of course, the protrusions are also for loosening of the needle hub 8. Also, the torque ring 5 is provided with a plurality of resilient arms 9 (9a-d) extending inwards from the torque ring 5, wherein the arms 9 together define a central space within which the device hub 4 is rotatable.

In other embodiments, the torque ring 5 may alternatively have other shapes than round/circular, such as square or some other n-gon shape, or a discontinuous shape such as C-shaped, as long as it allows for torque to be applied to the device hub 4.

The device hub 4 is provided with a plurality of abutment portions 10 in the form of protrusions configured to engage respective inner end portions of the respective arms 9 upon relative rotation between the device hub 4 and the torque ring 5 such that a momentum applied to the torque ring 5 is transferred to the device hub 4 via the engagement between the arms 9 and the abutment portions 10. In other embodiments, the abutment portions 10 may alternatively comprise recesses or portions of a different material with higher friction as compared to the material of the rest of the device hub 4. For example, a main material of the device hub 4 may be a thermoplastic material such as PP, i.e. Polypropylene, or PC, i.e. Polycarbonate, whereas the abutment portion may comprise a portion of a TPE material, i.e. Thermoplastic Elastomers, suitable for adhering to the main material. The arms and the abutment portions are configured so that the arms bend and slip over the abutment portions 10 upon application of momentum over a predetermined threshold level at rotation of the torque ring 5 in a tightening direction 11 for mounting of the needle hub 4 to the syringe barrel 3.

The torque ring 5 enables a user to grab the torque device 1 and apply momentum to the needle hub 8 with little effort and improved control. The device hub 4 holds the needle 2 in its central recess 6 while it is screwed onto the tip of the syringe barrel 3. The arms 9 transfer torque from the torque ring 5 to the needle hub 8 whilst limiting the maximum momentum transferable to the needle hub 8 upon tightening rotation by the resiliency of the arms 9 allowing them to slip past the abutment portions 10 once the torque applied exceeds a predetermined threshold level.

The predetermined threshold level is such that an appropriate connection is achieved between the syringe barrel 3 and the needle hub 8 to thereby secure the needle 2 to the syringe barrel 3 without tensioning the threaded connection too much or too little. Tensioning the connection too much would risk breaking the thread or parts of the syringe barrel 3 or needle hub 8. Tensioning the connection too little would lead to an increased risk of leakage or risk of unintentional separation of the needle hub 8 from the syringe barrel 3.

In the illustrated embodiment, the plurality of arms 9 comprises four arms 9a-d radially separated around the torque ring 5. By providing four arms 9a-d radially distributed around the torque ring 5, the arms 9a-d support the device hub 4 from four directions, thereby preventing the device hub 4 from leaving the central space upon operation of the torque device 1. In other embodiments, the number of arms 9 may be higher or lower as long as they fulfil the same function to hold the torque ring 5 positioned about the device hub 4, and to allow transfer of torque from the torque ring 5 to the device hub 4.

The device hub 4 is provided with a circumferential flange 12 extending radially outwards around the rotational axis 7 of the device hub, wherein the plurality of arms 9a-d engage the flange 12 from both axial sides of the flange 12. The provision of arms 9a-d engaging the flange 12 from both axial sides mitigates axial relative displacement between the torque ring 5 and the device hub 4 and assists the user in is operation of the torque device 1. In the illustrated embodiment, separate arms 9a-d extend on separate axial sides 13, 14 of the flange 12 but in other embodiments, one or more arms could each be provided with a split end with a respective recess that grabs the flange from both axial sides, for example as a U-shaped or Y-shaped end of the respective arm. Alternatively, the flange may comprise two spaced apart flanges with a recess in-between for receiving the arms such that the arms prevent relative axial movement between the device hub and the torque ring.

The device hub 4 is slightly larger than the central space defined by the arms 9a-d. The arms 9a-d of the torque ring 5 bend slightly when the device hub 4 is mounted in the torque ring 5. Hence, the arms 9a-d are normally biased towards the device hub 4, which ensures a proper fit between device hub 4 and torque ring 5 although the exact size of the device hub 4 may vary slightly. Further, since the arms 9a-d are normally biased, the power required for the arms 9a-d to pass the abutment portions 10 is more even throughout the relative rotational movement of the torque ring 5 around the device hub 4.

Two of the four arms - for reference called a first arm 9a and a second arm 9b - are provided on radially opposing sides of the device hub 4 with respect to each other, both on a first axial side 13 of the circumferential flange 12, as shown in fig. 4.

The plurality of arms 9 also comprises a third arm 9c and a fourth arm 9d provided on radially opposing sides of the device hub 4 with respect to each other, both on a second axial side 14 of the flange 12, said second axial side 14 being opposite the first axial side 13. The first arm 9a and second arm 9b together define a first pivot axis 15 for the flange 12 (to pivot about), as shown in figs. 1 and 2. Similarly, the third arm 9c and fourth arm 9d together define a second pivot axis 16 for the flange 12. Also, the first, second, third and fourth arms 9a-d are positioned such that the first pivot axis 15 and second pivot axis 16 are not parallel. The first and second arms 9a-b thus block movement of the flange 12 in a first axial direction along the rotational axis 7 of the torque device 1 whilst the third and fourth arms 9c-d block movement of the flange 12 in a second axial direction opposite the first axial direction. Since the first and second pivot axes 15, 16 are not parallel, any rotation of the device hub 4 around the first pivot axis 15 is prevented by the third and fourth arms 9c-d and vice versa. This provides for a very simple and robust technical solution allowing the device hub 4 to be easily inserted into the central space upon manufacturing of the torque device 1.

At least one of the arms 9a-d is attached to the torque ring 5 backward or forward of the normal 17 of the torque ring 5, with reference to the tightening direction 11 of the torque ring 5, said normal extending through the tip of the arm 9 as shown in fig. 2. In other words, said at least one of the arms 9a-d extends obliquely backward or forward of the normal 17.

By so directing the arm, the tip of the arm 9a-d moves more radially outward upon bending of the arm 9a-d as compared to if the arm 9a-d would be directed straight along the radial direction of the needle hub. The increased tendency of the tip to move radially outward upon being forced onto the abutment portions 10 at application of torque on the torque ring 5 provides for a more distinct click when the arm passes the abutment portions 10; This, since only a little relative rotation is required to move the tip of the arm 9a-d radially outward enough for the abutment portions 10 to be able to slide under and past the arm 9a-d.

At least some of the abutment portions 10 may comprise a leading surface 18 angled in the range of 20-130 degrees relative to the opening direction 19 of the respective arm at contact with the respective arm upon application of torque on the torque ring for tensioning of the needle to the syringe barrel. When the leading surface is so oriented, the tip of the arm is forced in it's opening direction by the leading surface upon application of increased torque for tensioning of the needle hub to the syringe barrel.

It should be noted that for all embodiments of the torque device, the torque device works just as well for cannulas as for needles and hence the terms are used interchangeably. Thus, the appended claims should also be interpreted as covering cannulas as alternative for needles wherever needles are mentioned. Also, it should be noted that the needle hub is usually handled with a needle cover 21 mounted on the needle hub in order to avoid contamination of the needle/cannula and also to avoid injuries caused by the needle/cannula. It is immaterial to the invention whether the torque device applies the torque to the needle hub directly or if it instead does it indirectly via the needle cover/cannula cover 21 attached to the needle hub. If the needle is handled without cover 21, the torque device is attached directly to the needle hub.

## Claims

1. A torque device (1) for mounting of a syringe needle (2) to a syringe barrel (3), said torque device comprising a device hub (4) and a torque ring (5),
wherein said device hub (4) is provided with a central recess (6) extending along a rotational axis (7) of the device hub (4),
wherein said central recess (6), is configured to receive and grab a needle hub (8) of the syringe needle (2), directly or via a needle cover (21) attached to the needle hub (8), and with the needle (2) extending along the rotational axis (7) of the device hub (4),
wherein the torque ring (5) is provided with a plurality of resilient arms (9) extending inwards from the torque ring (5), wherein the arms (9) together define a central space within which the device hub (4) is rotatable,
wherein the device hub (4) is provided with a plurality of abutment portions (10) configured to engage respective inner end portions of the respective arms (9) upon relative rotation between the device hub (4) and the torque ring (5) such that a momentum applied to the torque ring (5) is transferred to the device hub (4) via the engagement between the arms (9) and the abutment portions (10), wherein the arms (9) and the abutment portions (10) are configured so that the arms (9) bend and slip over the abutment portions (10) upon application of momentum over a predetermined threshold level at rotation of the torque ring (5) in a tightening direction (11) for mounting of the needle hub (4) to the syringe barrel (3).

2. A torque device (1) according to claim 1 wherein the plurality of arms (9) comprises at least three arms (9) radially separated around the torque ring (5).

3. A torque device (1) according to any one of the preceding claims wherein the device hub (4) is provided with a circumferential flange (12) extending radially outwards around the rotational axis (7) of the device hub (4),
wherein the plurality of arms (9) engages the flange (12) from both axial sides of the flange (12).

4. A torque device (1) according to any one of the preceding claims, wherein the device hub (4) is larger than the central space defined by the arms (9).

5. A torque device (1) according to any one of the preceding claims, wherein the plurality of arms (9) comprise
a first arm (9a) and a second arm (9b) provided on radially opposing sides of the device hub (4) with respect to each other, both on a first axial side (13) of the circumferential flange (12), and
a third arm (9c) and a fourth arm (9d) provided on radially opposing sides of the device hub (4) with respect to each other, both on a second axial side (14) of the flange (12), said second axial side (14) being opposite the first axial side (13),
wherein the first and second arms (9a, 9b) together define a first pivot axis (15),
wherein the third and fourth arms (9c, 9d) together define a second pivot axis (16), and
wherein the first, second, third and fourth arms (9a-d) are positioned such that the first pivot axis (15) and second pivot axis (16) are non-parallel.

6. A torque device (1) according to any one of the preceding claims wherein at least one of the arms (9) is attached to the torque ring (5) backward or forward of the normal (17) of the torque ring (5), with reference to the tightening direction of the torque ring 5, said normal through the tip of the arm (9).

7. A torque device (1) according to any one of the preceding claims, wherein at least some of the abutment portions (10) comprise a leading surface (18) angled in the range of 20-130 degrees relative to the opening direction (19) of the respective arm at contact with the respective arm upon application of torque on the torque ring for tensioning of the needle to the syringe.

## Patentansprüche

1. Drehmomentvorrichtung (1) zum Montieren einer Spritzennadel (2) an einem Spritzenzylinder (3), wobei die Drehmomentvorrichtung einen Vorrichtungsansatz (4) und einen Drehmomentring (5) umfasst,
wobei der Vorrichtungsansatz (4) mit einer mittleren Aussparung (6) versehen ist, die sich entlang einer Rotationsachse (7) des Vorrichtungsansatzes (4) erstreckt,
wobei die mittlere Aussparung (6) dazu ausgestaltet ist, einen Nadelansatz (8) der Spritzennadel (2) direkt oder über eine an dem Vorrichtungsansatz (8) angebrachte Nadelabdeckung (21) aufzunehmen und zu ergreifen, wobei sich die Nadel (2) entlang der Rotationsachse (7) des Vorrichtungsansatzes (4) erstreckt,
wobei der Drehmomentring (5) mit einer Vielzahl von federnden Armen (9) versehen ist, die sich von dem Drehmomentring (5) nach innen erstrecken, wobei die Arme (9) zusammen einen mittleren Raum definieren, in dem der Vorrichtungsansatz (4) drehbar ist,
wobei der Vorrichtungsansatz (4) mit einer Vielzahl von Anlageabschnitten (10) versehen ist, die dazu ausgestaltet sind, bei Relativdrehung zwischen dem Vorrichtungsansatz (4) und dem Drehmomentring (5) jeweilige innere Endabschnitte der jeweiligen Arme (9) in Eingriff zu nehmen, so dass ein auf den Drehmomentring (5) ausgeübtes Moment über den Eingriff zwischen den Armen (9) und den Anlageabschnitten (10) auf den Vorrichtungsansatz (4) übertragen wird, wobei die Arme (9) und die Anlageabschnitte (10) so ausgestaltet sind, dass sich die Arme (9) bei Ausübung eines Moments über einem vorbestimmten Schwellenniveau bei Drehung des Drehmomentrings (5) in eine Anziehrichtung (11) zum Montieren des Nadelansatzes (4) an den Spritzenzylinder (3) biegen und über die Anlageabschnitte (10) schlüpfen.

2. Drehmomentvorrichtung (1) nach Anspruch 1, wobei die Vielzahl von Armen (9) mindestens drei Arme (9) umfassen, die radial um den Drehmomentring (5) herum beabstandet sind.

3. Drehmomentvorrichtung (1) nach einem der vorhergehenden Ansprüche, wobei der Vorrichtungsansatz (4) mit einem Umfangsflansch (12) versehen ist, der sich um die Rotationsachse (7) des Vorrichtungsansatzes (4) radial nach außen erstreckt, wobei die Vielzahl von Armen (9) den Flansch (12) von beiden axialen Seiten des Flanschs (12) in Eingriff nehmen.

4. Drehmomentvorrichtung (1) nach einem der vorhergehenden Ansprüche, wobei der Vorrichtungsansatz (4) größer als der von den Armen (9) definierte mittlere Raum ist.

5. Drehmomentvorrichtung (1) nach einem der vorhergehenden Ansprüche, wobei die Vielzahl von Armen (9) Folgendes umfassen:
einen ersten Arm (9a) und einen zweiten Arm (9b), die bezüglich einander an radial gegenüberliegenden Seiten des Vorrichtungsansatzes (4) und beide an einer ersten axialen Seite (13) des Umfangsflanschs (12) vorgesehen sind, und
einen dritten Arm (9c) und einen vierten Arm (9d), die bezüglich einander an radial gegenüberliegenden Seiten des Vorrichtungsansatzes (4) und beide an einer zweiten axialen Seite (14) des Flanschs (12) vorgesehen sind, wobei die zweite axiale Seite (14) der ersten axialen Seite (13) gegenüberliegt,
wobei der erste und der zweite Arm (9a, 9b) zusammen eine erste Schwenkachse (15) definieren,
wobei der dritte und der vierte Arm (9c, 9d) zusammen eine zweite Schwenkachse (16) definieren und
wobei der erste, der zweite, der dritte und der vierte Arm (9a-d) so positioniert sind, dass die erste Schwenkachse (15) und die zweite Schwenkachse (16) nicht parallel sind.

6. Drehmomentvorrichtung (1) nach einem der vorhergehenden Ansprüche, wobei mindestens einer der Arme (9) bezüglich der Festziehrichtung des Drehmomentrings (5) hinter oder vor der Normalen (17) des Drehmomentrings (5) an dem Drehmomentring (5) angebracht ist, wobei die Normale durch die Spitze des Arms (9) verläuft.

7. Drehmomentvorrichtung (1) nach einem der vorhergehenden Ansprüche, wobei mindestens einer der Anlageabschnitte (10) eine vordere Fläche (18) umfasst, die in einem Bereich von 20 - 130 Grad bezüglich der Öffnungsrichtung (19) des jeweiligen Arms bei Kontakt mit dem jeweiligen Arm bei Ausübung eines Drehmoments auf den Drehmomentring zum Spannen der Nadel an der Spritze abgewinkelt ist.

## Revendications

1. Dispositif d'application de couple (1) pour la fixation d'une aiguille de seringue (2) à un corps de seringue (3), ledit dispositif d'application de couple comprenant un moyeu de dispositif (4) et une bague d'application de couple (5),
dans lequel ledit moyeu de dispositif (4) est pourvu d'un évidement central (6) s'étendant le long d'un axe de rotation (7) du moyeu de dispositif (4),
dans lequel ledit évidement central (6) est conçu pour recevoir et agripper un pavillon d'aiguille (8) de l'aiguille de seringue (2), directement ou par le biais d'un étui protecteur d'aiguille (21) attaché au pavillon d'aiguille (8), et de sorte que l'aiguille (2) s'étende le long de l'axe de rotation (7) du moyeu de dispositif (4),
dans lequel la bague d'application de couple (5) est pourvue d'une pluralité de bras élastiques (9) s'étendant vers l'intérieur à partir de la bague d'application de couple (5), les bras (9) définissant conjointement un espace central à l'intérieur duquel le moyeu de dispositif (4) peut tourner,
dans lequel le moyeu de dispositif (4) est pourvu d'une pluralité de parties de butée (10) conçues pour interagir avec des parties d'extrémité intérieures respectives des bras (9) respectifs lors d'une rotation relative entre le moyeu de dispositif (4) et la bague d'application de couple (5) de telle sorte qu'une impulsion imprimée à la bague d'application de couple (5) soit transférée au moyeu de dispositif (4) par le biais de l'interaction entre les bras (9) et les parties de butée (10), les bras (9) et les parties de butée (10) étant conçus de telle sorte que les bras (9) se courbent et glissent par-dessus les parties de butée (10) lors de l'application d'une impulsion supérieure à un niveau seuil prédéterminé au cours d'une rotation de la bague d'application de couple (5) dans une direction de serrage (11) pour la fixation du pavillon d'aiguille (4) au corps de seringue (3).

2. Dispositif d'application de couple (1) selon la revendication 1, dans lequel la pluralité de bras (9) comprend au moins trois bras (9) séparés radialement autour de la bague d'application de couple (5).

3. Dispositif d'application de couple (1) selon l'une quelconque des revendications précédentes, dans lequel le moyeu de dispositif (4) est pourvu d'une collerette circonférentielle (12) s'étendant radialement vers l'extérieur autour de l'axe de rotation (7) du moyeu de dispositif (4), la pluralité de bras (9) interagissant avec la collerette (12) depuis les deux côtés axiaux de la collerette (12).

4. Dispositif d'application de couple (1) selon l'une quelconque des revendications précédentes, dans lequel le moyeu de dispositif (4) est plus large que l'espace central défini par les bras (9).

5. Dispositif d'application de couple (1) selon l'une quelconque des revendications précédentes, dans lequel la pluralité de bras (9) comprend
un premier bras (9a) et un deuxième bras (9b) placés sur des côtés radialement opposés du moyeu de dispositif (4) l'un vis-à-vis de l'autre, tous deux sur un premier côté axial (13) de la collerette circonférentielle (12), et
un troisième bras (9c) et un quatrième bras (9d) placés sur des côtés radialement opposés du moyeu de dispositif (4) l'un vis-à-vis de l'autre, tous deux sur un second côté axial (14) de la collerette (12), ledit second côté axial (14) étant opposé au premier côté axial (13),
dans lequel les premier et deuxième bras (9a, 9b) définissent conjointement un premier axe de pivotement (15),
dans lequel les troisième et quatrième bras (9c, 9d) définissent conjointement un second axe de pivotement (16), et
dans lequel les premier, deuxième, troisième et quatrième bras (9a à d) sont positionnés de telle sorte que le premier axe de pivotement (15) et le second axe de pivotement (16) soient non parallèles.

6. Dispositif d'application de couple (1) selon l'une quelconque des revendications précédentes, dans lequel au moins un des bras (9) est attaché à la bague d'application de couple (5) en arrière ou en avant par rapport à la normale (17) de la bague d'application de couple (5), relativement à la direction de serrage de la bague d'application de couple (5), ladite normale passant par la terminaison du bras (9).

7. Dispositif d'application de couple (1) selon l'une quelconque des revendications précédentes, dans lequel au moins certaines des parties de butée (10) comprennent une surface d'attaque (18) inclinée de 20 à 130 degrés par rapport à la direction d'ouverture (19) du bras respectif lors de l'entrée en contact avec le bras respectif suite à l'application d'un couple sur la bague d'application de couple à des fins de serrage de l'aiguille sur la seringue.
